# EUROPEAN PATENT SPECIFICATION

(11) **EP 0 649 517 B1**
(45) Date of publication and mention of the grant of the patent: **27.08.1997**
(21) Application number: 92921187.8
(22) Date of filing: 08.07.1992
(51) Int. Cl.: G01J 5/20

(54) **MICROSTRUCTURE DESIGN FOR HIGH IR SENSITIVITY**
MIKROSTRUKTUR-ENTWURF FÜR HOHE IR - EMPFINDLICHKEIT
CONSTRUCTION DE MICROSTRUCTURE PERMETTANT D'OBTENIR UNE SENSIBILITE INFRAROUGE (IR) ELEVEE

(43) Date of publication of application: 26.04.1995
(73) Proprietor: HONEYWELL INC., Minneapolis Minnesota 55408 (US)
(72) Inventor: COLE, Barrett, E., Bloomington, MN 55344 (US)
(74) Representative: Fox-Male, Nicholas Vincent Humbert
(86) International application number: US9205699
(87) International publication number: WO9401743

(56) References cited:
- EP-A- 0 354 369
- EP-A- 0 417 845
- PATENT ABSTRACTS OF JAPAN vol. 16, no. 38 (E-1161) 30 January 1992 & JP-A-32 48 477
- PATENT ABSTRACTS OF JAPAN vol. 13, no. 387 (P-924) 28 August 1989 & JP-A-11 36 035

## Description

### FIELD OF THE INVENTION

The field of the invention is in a high sensitivity two-level microstructure infrared bolometer array which can produce absorptance levels of greater than 80% and also achieve high IR sensitivity over a wavelength range from 8-14 x 10⁻⁶m (8-14 microns).

### BACKGROUND AND SUMMARY OF THE INVENTION

European Patent Specification Publication No. EP-A-0354369 discloses a bolometer structure having an upper detector place connected to a lower silicon substrate by metal inter connectors.

The present invention provides microbridge infrared bolometer structure comprising a bolometer structure on a semiconductor substrate, said structure having a lower section on the surface of the substrate and a microbridge upper detector plane structure spaced from and immediately above the lower section; an infrared-reflective thin film metal coating on the surface of said lower section; said upper microbridge detector plane structure comprising a planar sandwich structure including a supporting dielectric thin film layer, and a thin film temperature responsive resistive element having first and second terminals; the structure characterised by downwardly extending dielectric leg portion means which are a downwardly extending continuation of said upper structure dielectric supporting said upper microbridge detector plane structure above said lower section so that a thermal isolation gap exists between said upper and lower sections; and electrically conductive paths included in said downwardly extending leg portion means connecting said first and second terminals to said lower section.

### BRIEF DESCRIPTION OF THE DRAWINGS

Figures 1 and 2 are front and top views of a microstructure design according to the invention.

Figure 3 is a graphical plot of overall absorptance vs. wavelength of the device over a selected wavelength including 8-14 x 10⁻⁶m (8-14 microns).

Figure 4 shows graphically the transmittance, absorption and reflectance of the resistive layer.

Figure 5 shows graphically absorption vs. air gap thickness.

Figure 6 shows absorption of entire structure vs. metal absorber thickness.

Figure 7 shows measured optical properties of Si₃ N₄.

### DESCRIPTION

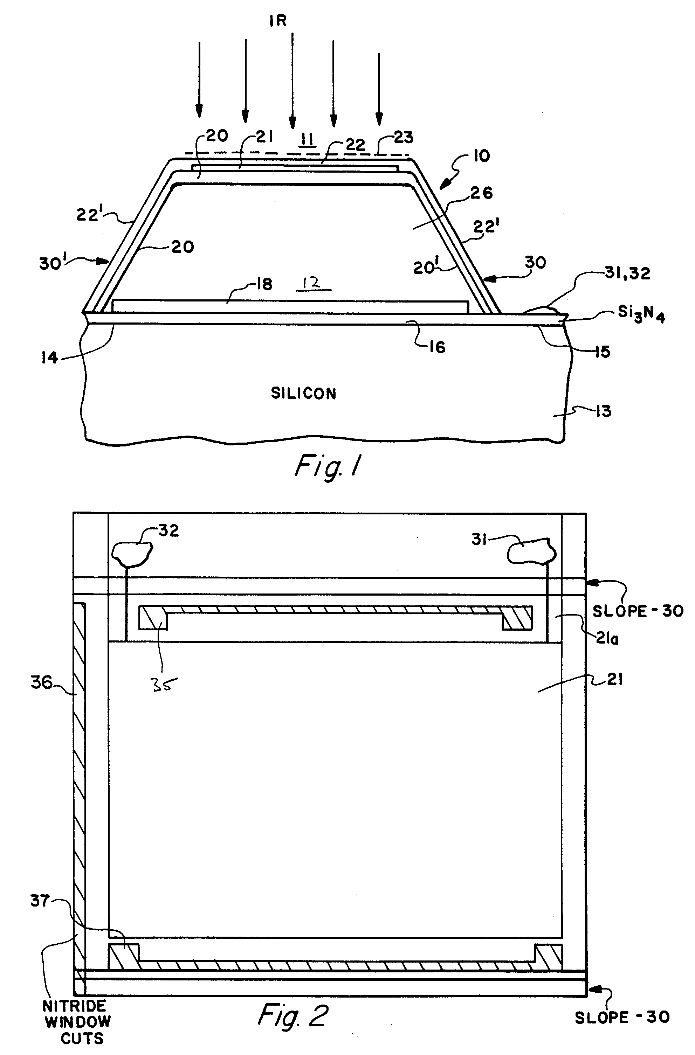

A cross section view of the two-level microbridge bolometer pixel 10 is shown in Figure 1. The device 10 has two levels, an elevated microbridge detector level 11 and a lower level 12. The lower level has a flat surfaced semiconductor substrate 13, such as a single crystal silicon substrate. The surface 14 of the substrate has fabricated thereon conventional components of an integrated circuit 15 such as diodes, bus lines, connections and contact pads (not specifically shown), the fabrication following conventional silicon IC technology. The IC is coated with a protective layer of silicon nitride 16.

The elevated detector level 11 includes a silicon nitride layer 20, a thin film resistive layer 21, preferably a vanadium or titanium oxide (such as V₂O₃, TiOₓ, VOₓ), i.e. ABₓ a silicon nitride layer 22 over the layers 20 and 21 and an IR absorber coating 23 over the silicon nitride layer 22. The thin absorber coating (approximately 20A thick) may be of a nickel iron alloy, often called permalloy. Downwardly extending silicon nitride layers 20' and 22' deposited at the same time as layers 20 and 22 during the fabrication make up the sloping supports 30 for the elevated detector level. The cavity or gap 26 (approximately 1-2 x 10⁻⁶m or 1-2 microns high) between the two levels is ambient atmosphere. During the fabrication process, however, the cavity 26 was originally filled with a previously deposited layer of easily dissolvable glass or other dissolvable material until the layers 20, 20', 22 and 22' were deposited. Some other easily dissolvable materials are quartz, polyimide and resist. Subsequently in the process the glass was dissolved out to provide the thermal isolation cavity or air gap (i.e., the air gap actually may be in operation, a vacuum gap). In Figure 1 the horizontal dimension, as shown, is greatly foreshortened. That is, the height of Figure 1 is exaggerated in the drawing compared to the length in order to show the details of the invention.

Figure 2 is a top plan view of the elevated detector level 11. This drawing is made as though the overlying absorber coating 23 and the upper silicon nitride layer 22 are transparent so that the resistive thin film layer 21 can be shown. In one preferred embodiment the material for the resistive layer 21 is a vanadium oxide, preferably V₂O₃. Vanadium oxides have very strong changes in resistance with temperature allowing high sensitivity microbolometer operation. It also has a low reflectance to IR in the 8-14 x 10⁻⁶m (8-14 micron) range. In the preferred embodiment at this time the V₂O₃ is operated in its semiconductor phase. Its deposition is preferably by the process of ion beam sputter which permits the deposition of very thin layers such as 5-7.5 x 10⁻⁵m (50-75nm). This material was thus selected for its low IR reflectance together with a relatively high temperature coefficient of resistance (TCR). The ends of the resistive paths 21a and 21b are continued down the slope area 30 embedded in 20' and 22' to make electrical contact with contact pads 31 and 32 on the lower level.

Figure 2 also shows nitride window cuts 35, 36 and 37 which are opened through the silicon nitride layers 20 and 22 to provide access to the phos-glass beneath for dissolving it from beneath the detector plane. The sloping supports may be of the necessary length to provide adequate support and thermal isolation for the upper level 11.

Although the description is basically in terms of individual detector pixels, the invention is directed for use to an x, y array assembly of adjoining pixels forming an imaging or mosaic detector array. Each pixel assembly may cover an area about 5x10⁻⁵m (50 microns) on a side, as an example.

Referring again to Figure 1 a sequence of fabrication steps for the upper level is described. Following the deposition of the silicon nitride layer 16 in fabricating the lower level 12, a thin film layer 18 of reflective material, such as a metal film like Pt or Au, is deposited. The construction of the upper level can then commence. The detectors presently being described are intended for use in the 8-14 x 10⁻⁶m (8-14 micron) IR wavelength. The reflective layer 18 is on the lower plane 12. The vertical distance between reflective layer 18 and upper level 11 is chosen so the reflected IR from layer 18 returned upwardly has interference properties such that significant absorption is achieved for a wide range of wavelengths 8-14 x 10⁻⁶m (8-14 microns) and air gap spacing between the reflector and the detector structure.

A layer of phos-glass or other easily soluble material in the range of about 1-2 x 10⁻⁶m (1-2 microns) thick is deposited and the slopes 30 and 30' are thoroughly rounded to eliminate slope coverage problems. The upper level silicon nitride base layer 20 is then deposited, the resistive film 21 is deposited, connections down the slope to lower plane contact pads are made, and a silicon nitride passivation layer 22 covers the layers 21 and 20. A thin metal absorber coating 23 (about 1.5-4 x 10⁻⁵m or 15-40A) is deposited on top of the upper level. The slots 35, 36 and 37, earlier mentioned are made and the phos-glass is dissolved from beneath the detector plane. As earlier described, by depositing Pt, Au or other reflecting thin film 18 on the substrate before the stack is formed, it is possible to reflect transmitted radiation reaching the reflecting film back to the absorber coating.

The optical properties of the total structure are achieved by careful selection of optical materials with the proper optical and electrical properties. The top film must reflect little radiation and generally transmit a significant percentage of the non-absorbed radiation through to the reflected light at a nodal position in the film determined by the air gap distance. An additional constraint on this absorbing film is that to be compatible with the total structure, the absorbing material must be very thin (and hence have a low mass).

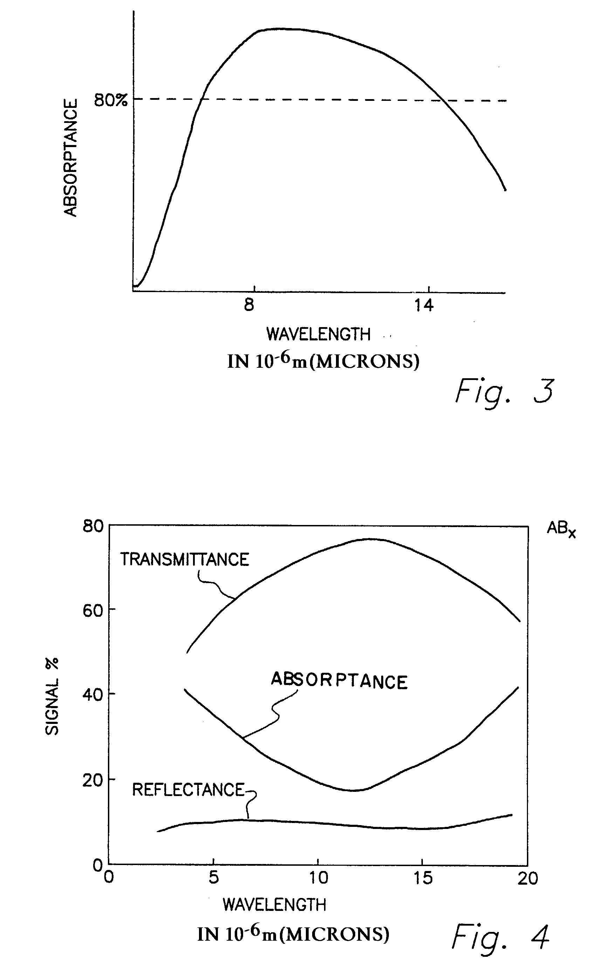

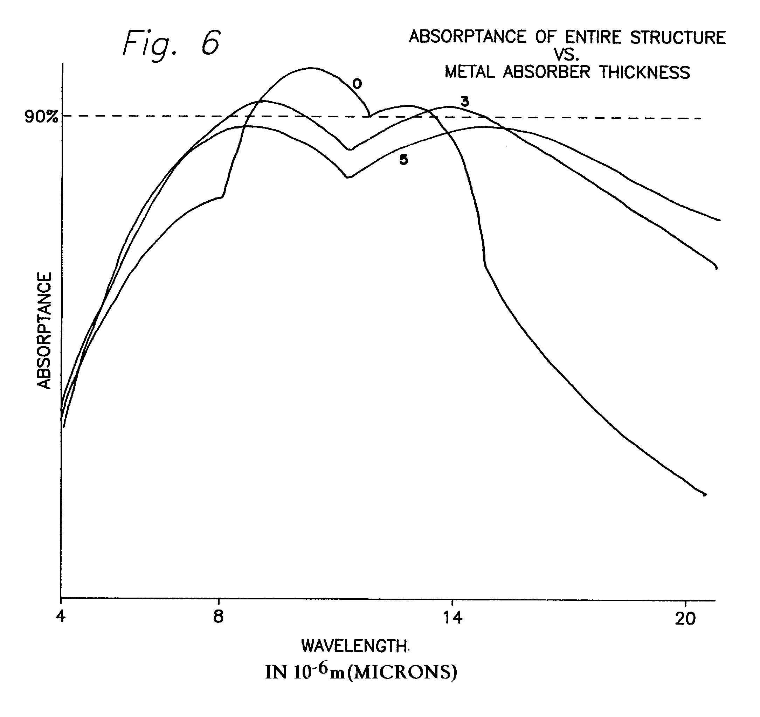

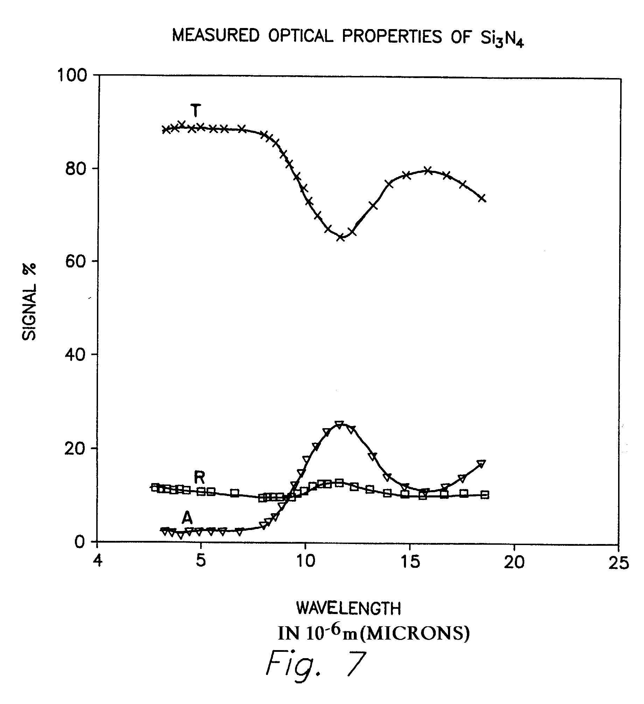

To optimize the absorption in the structure, the thickness of all the absorbing layers and the air gap distance must be controlled. The absorbing films in the present device consist of ABx, SIN, and the thin absorbing metal described above. In practice, the ABx and SIN nitride thicknesses are chosen by electrical and physical requirements. Both have absorption levels ranging from 10-20% in the spectral region of interest (Figures 4 and 7). A combination of these materials produces an absorption of no more than about 30% in the 8-14 x 10⁻⁶m (8-14 micron) region. This absorption level is very close to ideal, however, for use with a Pt reflective layer and an air gap which intensifies the field in the absorbing film, it is possible to achieve absorptances in excess of 80% (Figure 3) in this configuration. The use of a thin absorbing metal which in the standard design provides 50% absorption, here is used to fine tune the absorption for maximum effect. Figure 6 shows the small absorption improvements that can be achieved by using this metal film.

In this two-level structure, the low thermal mass structure 11 is separated from the Pt/substrate layer by an air gap. The interference properties of this reflected radiation are such that significant absorption is achieved for a wide range of wavelengths and air gap spacing between the Pt reflector and the detector structure.

For this optical interference to occur in the detector, it is necessary to avoid other films in the detector structure which reflect IR. The use of ABₓ which has both a high TCR and a low IR reflectance (Figure 4) ideally meets these requirements. Thus the merging of this absorption phenomenon into a detector structure which has a detector material processing both a high TCR and low reflectance permits this interference effect to occur.

There is a substantial degree of variability of detector absorptance with air gap in the structure. Referring to the table below which shows wavelength in nanometers in the left column vs. air gap in microns across the top it can be seen that with an air gap of only 5 x 10⁻⁷m (.5 micron) the detector absorptance varies widely with wavelength and is not very high. With air gaps of 1-2 x 10⁻⁶m (1-2 microns) and especially at 1.5 x 10⁻⁶m (1.5 microns) the absorptance is relatively high across the desired wavelength spread.

**TABLE 1 -**

| DETECTOR ABSORPTANCE | | | | | |
|---|---|---|---|---|---|
| | Air Gap x 10⁻⁶m (Microns) | | | | |
| Wavelength x 10⁻⁶ metres (NM) | .5 | .75 | 1.0 | 1.5 | 2.0 |
| 8 (8000) | .89 | .91 | .9 | .84 | .76 |
| 9 (9000) | .84 | .88 | .89 | .86 | .81 |
| 10 (10000) | .76 | .82 | .84 | .84 | .82 |
| 11 (11000) | .69 | .77 | .8 | .82 | .82 |
| 12 (12000) | .66 | .74 | .79 | .83 | .84 |
| 13 (13000) | .64 | .78 | .85 | .93 | .94 |
| 14 (14000) | .56 | .72 | .83 | .95 | .98 |
| 15 (15000) | .47 | .64 | .77 | .92 | .99 |

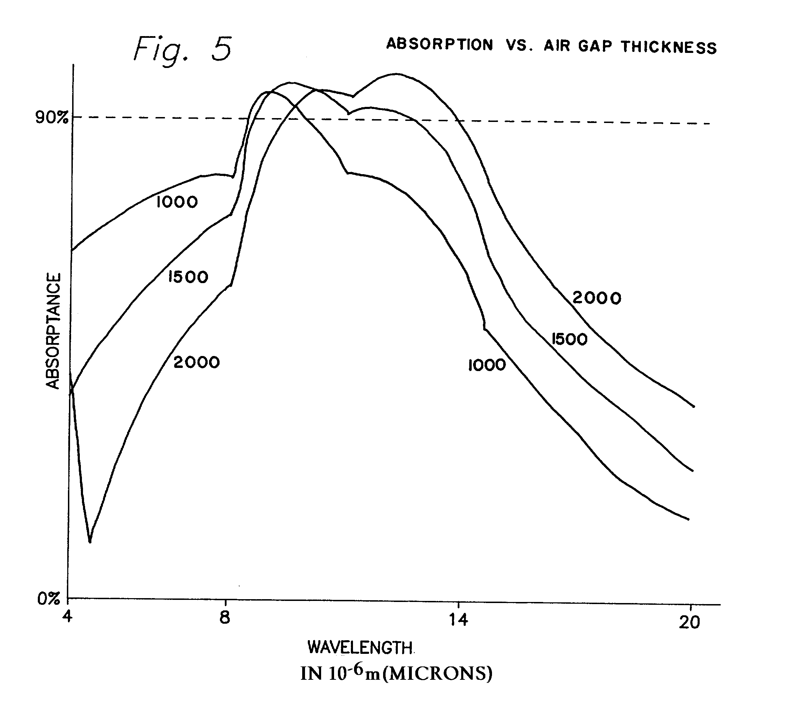

The effect of gap thickness on the absorptance vs. wavelength in the regions of interest are further displayed graphically in Figure 5. It can be seen in the curve of 1.5 x 10⁻⁶m (1.5 microns) gap thickness that at 8 x 10⁻⁶m (8 microns) the absorptance of the structure is climbing rapidly towards 90% and more, and that it remains relatively high out to about 14 microns. The curve for a gap of 2 x 10⁻⁶m (2 microns) shows that at IR wavelengths of 1.4 x 10⁻⁵m (14microns) the absorptance is better and well above 90%. In measuring the data for Figure 5 the absorber film 23 was not included in the stack structure.

Referring now to Figure 6 there is shown graphically how the overall absorptance of the film structure varies across the IR wavelength of 8-14 x 10⁻⁶m (8-14 microns) as the thickness of the metal absorber film is increased to 3 x 10⁻⁹m (3nm) and 5 x 10⁻⁹m (5nm). In this film stack design the Si₃N₄ layer 22 is 2.5 x 10⁻⁷m (100nm), the resistive film 21 is 7.5 x 10⁻⁸m (75nm) and the Si₃N₄ film 20 is 1 x 10⁻⁷m (100nm) with an air gap of 1.5 x 10⁻⁶m (1.5 microns) and a reflective Pt layer 18 5 x 10⁻⁸m (50nm). This curve for 3 x 10⁻⁹m (3nm) shows absorptance > 90% between 8 and 14 x 10⁻⁶m (8 and 14 microns).

The measured optical properties of reflectance R, transmissivity T, and absorptance A of the silicon nitride layers 20 and 22 (8 x 10⁻⁸m or otherwise 800 thick) are shown in Figure 7 with percent of signal shown on the ordinate axis and IR wavelength along the abscissa. It can be seen that the transmissivity at 8 x 10⁻⁶m (8 microns), about 90, and 1.4 x 10⁻⁵m (14 microns), about 80, is quite high and that the reflectance R at both 8 x 10⁻⁶m (8 microns) and 1.4 x 10⁻⁵m (14 microns) is well under ten.

## Claims

1. A microbridge infrared bolometer structure comprising a bolometer structure on a semiconductor substrate (13), said structure having a lower section on the surface of the substrate and a microbridge upper detector plane structure (11) spaced from and immediately above the lower section; an infrared-reflective thin film metal coating (18) on the surface (14) of said lower section; said upper microbridge detector plane structure comprising a planar sandwich structure including a supporting dielectric thin film layer (20), and a thin film temperature responsive resistive element (21) having first and second terminals;
the structure characterised by downwardly extending dielectric leg portion means (30) which are a downwardly extending continuation of said upper structure dielectric supporting said upper microbridge detector plane structure above said lower section so that a thermal isolation gap exists between said upper and lower sections; and
electrically conductive paths (21a, 21b) included in said downwardly extending leg portion means connecting said first and second terminals to said lower section.

2. A microbridge structure according to claim 1 wherein said reflective thin film metal coating (18) is selected from the group consisting of Au, Pt, and Al.

3. A microbridge structure according to claim 1 or 2 wherein said dielectric (20) is of silicon nitride.

4. A microbridge structure according to any of claims 1 to 3 wherein said thin film resistive element (21) is selected from the group consisting of vanadium oxide and titanium oxide.

5. A microbridge structure according to any preceding claim wherein said thin film resistive element (21) is V₂O₃.

6. A microbridge structure according to claim 1 wherein said gap between said lower section (12) and said upper detector structure (11) is in the range of about 1-2 x 10⁻⁶m (1-2 microns).

7. A microbridge structure according to any preceding claim wherein the coating (18) is about 5 x 10⁻⁸m (50nm) in thickness.

8. A microbridge structure according to any preceding claim and further comprising, in said planar sandwich structure, a second dielectric thin film layer (22) and a thin film absorber layer (23).

9. A microbridge structure according to any preceding claim wherein the first dielectric layer (20) is on the order of 100nm in thickness and the second dielectric layer (22) is on the order of 2.5 x 10⁻⁷m (250nm) in thickness.

10. A microbridge structure according to claim 8 or 9 wherein the resistive element film (21) is on the order of 5-7 x 10⁻⁸m (50-75nm) in thickness.

11. A microbridge structure according to any of claims 8 to 10 wherein the absorber layer (23) is on the order of 3 x 10⁻⁸m (30nm) in thickness.

## Patentansprüche

1. Mikrobrückenstruktur eines Infrarot-Bolometers mit:
einer auf einem Substrat (13) vorgesehenen Bolometerstruktur, welche ein Unterteil auf der Substratoberfläche sowie eine obere Mikrobrücken-Detektorebenen-Struktur (11) im Abstand von und unmittelbar über dem Unterteil aufweist;
einer IR-reflektierenden Dünnfilm-Metallschicht (18) auf der Oberfläche (14) des Unterteils; wobei
die obere Mikrobrücken-Detektorebenen-Struktur eine ebene Sandwich-Struktur mit einer tragenden dielektrischen Dünnschicht (20) sowie einem temperaturempfindlichen Dünnschicht-Widerstandselement (21) mit ersten und zweiten Anschlüssen aufweist;
**gekennzeichnet durch** sich nach unten erstreckende dielektrische Fußteile (30) der Struktur, welche eine sich nach unten erstreckende Fortsetzung der oberen dielektrischen Struktur sind und die obere Mikrobrücken-Detektorebenen-Struktur oberhalb des Unterteils derart halten, daß ein thermisch isolierender Spalt zwischen Ober- und Unterteil besteht; und
elektrische Leiterpfade (21a, 21b) in den sich nach unten erstreckenden Fußteilen, um die ersten und zweiten Anschlüsse mit dem Unterteil zu verbinden.

2. Mikrobrückenstruktur nach Anspruch 1, bei der die reflektierende Dünnfilm-Metallschicht (18) aus einem Material der Gruppe bestehend aus Gold, Platin und Aluminium ausgewählt ist.

3. Mikrobrückenstruktur nach Anspruch 1 oder 2, bei der das Dielektrikum (20) aus Siliziumnitrid besteht.

4. Mikrobrückenstruktur nach einem der Ansprüche 1 bis 3, bei der das Dünnschicht-Widerstandselement (21) aus einem Material der Gruppe bestehend aus Vanadiumoxyd und Titanoxyd ausgewählt ist.

5. Mikrobrückenstruktur nach einem vorangehenden Anspruch, bei der das Dünnschicht-Widerstandselement (21) aus V₂O₃ besteht.

6. Mikrobrückenstruktur nach Anspruch 1, bei der der Spalt zwischen dem Unterteil (12) und der oberen Detektorstruktur (11) 1 bis 2 x 10⁻⁶m (1 bis 2 Mikron) breit ist.

7. Mikrobrückenstruktur nach einem vorangehenden Anspruch, bei der die Schicht (18) eine Dicke von etwa 5 x 10⁻⁸m (50nm) hat.

8. Mikrobrückenstruktur nach einem vorangehenden Anspruch, welche in der ebenen Sandwich-Struktur eine zweite dielektrische Dünnfilmschicht (22) sowie eine Dünnfilm-Absorberschicht (23) aufweist.

9. Mikrobrückenstruktur nach einem vorangehenden Anspruch, bei der die erste dielektrische Schicht (20) eine Dicke in der Größenordnung von 100nm und die zweite dielektrische Schicht (22) eine Dicke in der Größenordnung von 2,5 x 10⁻⁷m (250nm) hat.

10. Mikrobrückenstruktur nach Anspruch 8 oder 9, bei der das Widerstands-Dünnfilmelement eine Dicke in der Größenordnung von 5 bis 7 x 10⁻⁸m (50 bis 75nm) aufweist.

11. Mikrobrückenstruktur nach einem der Ansprüche 8 bis 10, bei der die Absorberschicht (22) eine Dicke in der Größenordnung von 3 x 10⁻⁸m (30nm) hat.

## Revendications

1. Structure de bolomètre infrarouge à micro-jonction comprenant une structure de bolomètre sur un substrat semi-conducteur (13), ladite structure présentant une section inférieure sur la surface du substrat et une structure plane de détecteur supérieure à micro-jonction (11) espacée de la section inférieure et située immédiatement au-dessus de celle-ci; un revêtement métallique en film mince réfléchissant les infrarouges (18) sur la surface (14) de ladite section inférieure; ladite structure plane de détecteur à micro-jonction supérieure comprenant une structure en sandwich plane incluant une couche en film mince de diélectrique de support (20), et un élément résistif en film mince sensible à la température (21) comportant des première et seconde bornes,
la structure étant caractérisée par des moyens formant des parties en pied diélectriques s'étendant vers le bas (30) qui sont un prolongement, s'étendant vers le bas, dudit diélectrique de la structure supérieure supportant ladite structure plane de détecteur à micro-jonction supérieure au-dessus de ladite section inférieure, de sorte qu'un interstice d'isolation thermique existe entre lesdites sections supérieure et inférieure, et
des trajets électriquement conducteurs (21a, 21b) intégrés dans lesdits moyens formant parties en pied s'étendant vers le bas, reliant lesdites première et seconde bornes à ladite section inférieure.

2. Structure à micro-jonction selon la revendication 1, dans laquelle ledit revêtement métallique en film mince réfléchissant (18) est sélectionné parmi le groupe constitué de l'Au, du Pt, et de l'Al.

3. Structure à micro-jonction selon la revendication 1 ou 2, dans laquelle ledit diélectrique (20) est du nitrure de silicium.

4. Structure à micro-jonction selon l'une quelconque des revendications 1 à 3, dans laquelle ledit élément résistif en film mince (21) est sélectionné parmi le groupe constitué de l'oxyde de vanadium et de l'oxyde de titane.

5. Structure à micro-jonction selon l'une quelconque des revendications précédentes, dans laquelle ledit élément résistif en film mince (21) est du V₂O₃.

6. Structure à micro-jonction selon la revendication 1, dans laquelle ledit interstice entre ladite section inférieure (12) et ladite structure de détecteur supérieure (11) est dans la plage d'environ 1 à 2 x 10⁻⁶m (1 à 2 microns).

7. Structure à micro-jonction selon l'une quelconque des revendications précédentes, dans laquelle le revêtement (18) a une épaisseur d'environ 5 x 10⁻⁸ m (50 nm).

8. Structure à micro-jonction selon l'une quelconque des revendications précédentes et comprenant en outre, dans ladite structure en sandwich plane, une seconde couche en film mince de diélectrique (22) et une couche d'élément d'absorption en film mince (23).

9. Structure à micro-jonction selon l'une quelconque des revendications précédentes, dans laquelle la première couche de diélectrique (20) a une épaisseur de l'ordre de 100 nm et la seconde couche de diélectrique (22) a une épaisseur de l'ordre de 2,5 x 10⁻⁷m (250 nm).

10. Structure à micro-jonction selon la revendication 8 ou 9, dans laquelle le film d'élément résistif (21) a une épaisseur de l'ordre de 5 à 7 x 10⁻⁸m (50 à 75 nm).

11. Structure à micro-jonction selon l'une quelconque des revendications 8 à 10, dans laquelle la couche d'élément d'absorption (23) a une épaisseur de l'ordre de 3 x 10⁻⁸m (30 nm).
